# EUROPEAN PATENT SPECIFICATION

(11) **EP 0 750 849 B1**
(45) Date of publication and mention of the grant of the patent: **04.09.2002**
(21) Application number: 96108448.0
(22) Date of filing: 28.05.1996
(51) Int. Cl.: A23L 1/211, A61K 47/24, A23J 7/00, A61K 7/16

(54) **Taste modifier**
Geschmacksveränderungsmittel
Modificateur du goût

(30) Priority: 06.06.1995 JP 13925895
(43) Date of publication of application: 02.01.1997
(73) Proprietor: KAO CORPORATION, Chuo-ku, Tokyo (JP)
(72) Inventor: Katsuragi, Yoshihisa, c/o Kao Corp., Res. Lab., Kashima-gun, Ibaragi (JP); Umeda, Tomoshige, c/o Kao Corp., Res. Lab., Kashima-gun, Ibaragi (JP)
(74) Representative: Kraus, Walter, Dr.

(56) References cited:
- EP-A- 0 631 787
- EP-A- 0 716 811
- WO-A-94/25006
- PATENT ABSTRACTS OF JAPAN vol. 016, no. 295 (C-0957), 30 June 1992 & JP-A-04 079858 (KAO CORP), 13 March 1992,
- PATENT ABSTRACTS OF JAPAN vol. 018, no. 215 (C-1191), 18 April 1994 & JP-A-06 014711 (KAO CORP), 25 January 1994,
- DATABASE WPI Section Ch, Week 7912 Derwent Publications Ltd., London, GB; Class A96, AN 79-23038B XP002015150 & JP-A-54 020 142 (SUN STAR HAMIGAKI K) , 15 February 1979
- DATABASE WPI Section Ch, Week 9546 Derwent Publications Ltd., London, GB; Class A96, AN 95-355195 XP002015151 & JP-A-07 242 568 (EISAI CO LTD) , 19 September 1995

## Description

### FIELD OF THE INVENTION

This invention relates to a taste modifier which is capable of effectively improving the aftertaste when added to medicinal compositions for oral use, foods or cosmetics having unpleasant tastes (for example, bitterness, pungency, puckery taste, tang, astringency).

### BACKGROUND OF THE INVENTION

Tastes can be roughly classified into five fundamental ones (i.e., sweetness, saltiness, sourness, body and bitterness). In addition, there are some unpleasant tastes (for example, pungency, puckery taste, tang, astringency). In the field of pharmacy, in particular, most of drugs have bitter, tang or astringent taste, as the proverb says "Good medicine tastes bitter". Accordingly, it is an important problem in the drug manufacturing processes to mask the bitterness, etc.

In the drug industry, it has been the main practice to mask unpleasant tastes such as bitterness by sugar-coating (in the case of tablets), or sugar-coating or film-coating with the use of polymers (in the case of tablets, granules and fine granules). In the case of solid preparations in general, attempts have been also made to mask unpleasant tastes by microencapsulation, with the use of inclusion compounds (JP-A-3-236316; the term "JP-A" as used herein means an "unexamined published Japanese patent application"), or insolubilization of drugs via chemical modification. However, each of these methods suffers from some problems, for example, the unpleasant tastes such as bitterness cannot be completely masked thereby, a complicated procedure is required therefor, or the application range thereof is restricted to particular drugs. The problem of relieving unpleasant tastes is further serious in the case of liquid drugs. Since no coating is applicable to liquid preparations, attempts have been made to mask unpleasant tastes such as bitterness by blending sugars or organic acids at high concentration or adding flavors. However, each of these methods fails to give any complete masking effect. Liquid drugs and dry syrups to be dissolved before using are frequently administered, in particular, to infants and children who cannot well take solid drugs such as tablets and granules. Thus it is a serious problem in liquid drugs to mask unpleasant tastes such as bitterness.

Regarding foods, there arises the problem of bitterness in various foods, for example, the offensive tastes caused mainly by the bitterness of peptides and amino acids obtained from protein hydrolyzates and the bitterness of fruit juices. With the recent tendency toward healthful diets, there have been marketed a number of health foods of various types. Among these products, extracts originating in plants (for example, gymnemic acid, aloe) show intense bitterness. It is essentially required in many cases to eliminate offensive tastes from foods, since the qualities of the foods per se are deteriorated thereby. Examples of the existing methods for eliminating offensive taste components comprising mainly bitter components from foods include a method wherein an adsorbent is used (U.S. Patent 4,282,264 corresponding to JP-A-55-108254, JP-A-60-91969), one wherein an inclusion compound is used (JP-A-61-40260, JP-A-2-283246), one wherein a sweetener is added (JP-A-60-9774) and one wherein bitter components such as peptides are decomposed with enzymes and thus eliminated (JP-A-2-207768). A method of suppressing bitter taste in oral preparations comprising adding an acidic phospholipid or an acidic lysophospholipid to the oral preparations is known from EP-A-0 631 787.
WO 94/25006 discloses taste-masking of pharmaceutical compositions and methods of making the same, comprising mixing to the drug a lipid mixture and an emulsifier or a surfactant. Further, it has been a practice to modify food materials by preliminarily removing tissues containing offensive taste components such as bitter components therefrom. However, these methods each suffers from some problems, for example, the bitterness cannot be completely suppressed thereby or the taste of a food is changed thereby.

In some foods, pungency is essentially required for achieving a characteristic stimulus and flavor, together with body, etc. It is widely known that pungent components generally exert some effects on foods. For example, the pungent components of mustard retard deterioration of the qualities of meats and fishes, thus keeping them from going bad. It is also well known that the effects of these pungent components on human body include appetizing and the enhancement of physiological activities (elevating bodily temperature, promoting perspiration, etc.)

However, use of a pungent component is disturbed by its strong stimulus, when it is employed in a large amount in order to give a favorable flavor or to prevent foods from going bad, or it is taken in a large amount in order to enhance physiological effects for controlling the condition of health.

Regarding cosmetics, it is preferable that no component shows any unpleasant taste (for example, bitterness, tang, astringency) particularly in lotions, mouse wash agents, tooth pastes, etc. to be applied to the face or the oral cavity. However, some surfactants and flavors employed in these products show bitterness, etc., which restricts the type and content of such a component at using. The conventional method for relieving bitterness, etc. comprises adding sweeteners or specific flavors. However, there is a problem that this method exerts only an unsatisfactory effect on a component with an intense bitterness, etc.

Accordingly, an object of the present invention is to provide a taste modifier, which exerts an excellent taste modifying effect on drugs, foods or cosmetics containing offensive taste components (for example, bitter components) while keeping a high stability and a high safety and causing no change in the taste in the case of a food. Also, the present invention provides medicinal compositions for oral use, foods or cosmetics containing the above taste modifier.

Under these circumstances, the present inventors have conducted extensive studies. As a result, they have successfully found out that, when combined with a surfactant, an acidic phospholipid or a lyso form thereof can be uniformly dispersed in water in a stable state and largely contribute to the modification of taste, thus completing the present invention.

### SUMMARY OF THE INVENTION

Accordingly, the present invention provides a taste modifier which comprises the following components (A) and (B), as defined in claim 1.

### DETAILED DESCRIPTION OF THE INVENTION

The acidic phospholipid or its lyso form to be used as the component (A) in the present invention can be obtained from natural substances. Alternatively, they can be chemically synthesized. Among all, it is preferable to use a phosphatidic acid or a lysophosphatidic acid therefor.

The acidic phospholipid or its lyso form employed as the component (A) can be extracted and separated from various animal organs and plant tissues including soybean, yolk and wheat germ. The extraction method is exemplified by one with the use of organic solvents by taking advantage of a difference in polarity. On the other hand, the separation method is exemplified by the adsorption by a silica gel column followed by elution with an organic solvent.

To obtain an acidic phospholipid or its lyso form, it is also possible that a phospholipid analogue, which has been obtained by extracting, separating and purifying, is processed by chemical modification or enzymatic treatment. Particular examples thereof include a method for obtaining a phosphatidic acid wherein phosphatidylcholine, which is a neutral phospholipid, is hydrolyzed with the use of phospholipase D originating in oilseeds (U.S. Patent 5,183,750 corresponding to JP-A-2-312552) or plants such as cabbage or rice bran or phospholipase D produced by a microorganism (U.S. Patent 5,183,750 corresponding to JP-A-2-312551); a method wherein the phosphate group of phosphatidylcholine is subjected to ester interchange by using phospholipase D to thereby give phosphatidylglycerol (JP-A-3-22991); and a method wherein the concentration of a phosphatidic acid is elevated with the use of an organic solvent (for example, ethanol) by taking advantage of the polarity of a phospholipid component.

It is also possible to use an acidic phospholipid or a lyso form thereof obtained by chemical synthesis. For example, use can be made of those obtained by converting diglycerides or monoglycerides into phosphates.

Examples of the acidic phospholipids and lyso forms thereof obtained by the above-mentioned chemical synthesis methods include bisphosphatidic acid, bisphosphatidyl monophosphatidic acid and bisphosphatidyl lysophosphatidic acid. It is also possible to use hydrogenated phosphatidic acids and hydrogenated lysophosphatidic acids therefor.

As described above, the acidic phospholipid or its lyso form to be used as the component (A) in the present invention is obtained in the form of a lipid mixture containing the acidic phospholipid or its lyso form from a natural substance or by chemical synthesis. Although the lipid mixture may be used as such, it is preferable to elevate the concentration of the phospholipid or its lyso form in the lipid mixture, since an improved taste modifying effect can be thus established.

The lipid mixture contains acidic phospholipids such as phosphatidylserine, phosphatidylinositol, phosphatidylglycerol and cardiolipin or lyso forms thereof; neutral phospholipids such as phosphatidylcholine and phosphatidylethanolamine or lyso forms thereof; neutral lipids such as triglycerides, diglycerides and monoglycerides, fatty acids, sterol lipids, glycolipids, etc. The component (A) may also contain these substances.

Either one of these acidic phospholipids and lyso forms or a combination thereof may be used as the component (A).

The content of the acidic phospholipid or its lyso form of the component (A) in the taste modifier ranges from 1 to 99 % (by weight, the same will apply hereinafter unless otherwise noted), still preferably from 15 to 99 %. When a lipid mixture is used as the component (A), the content of the phosphatidic acid or its lyso form in the lipid mixture is preferably at least 5 %, still preferably at least 20 % and particularly preferably from 50 to 99 %, since an excellent taste modifying effect can be thus achieved.

The concentration of the acidic phospholipid or its lyso form in the lipid mixture can be elevated by the enzymatic decomposition or solvent fractionation of the lipid mixture containing the acidic phospholipid or its lyso form followed by treatment with acetone, membrane separation, etc.

The surfactant to be used as the component (B) in the present invention is chosen among esters of mono- or diglycerides with polycarboxylic acids.

These esters of mono- or diglycerides with polycarboxylic acids can be prepared by reacting mono- or diglycerides with polycarboxylic acids or reactive derivatives thereof.

Preferable examples of the fatty acid residues constituting the mono- or diglycerides are saturated or unsaturated fatty acid residues carrying 8 to 22 carbon atoms, still preferably 12 to 18 carbon atoms, though the present invention is not restricted thereto. Particularly preferable examples of the fatty acid residues constituting the mono- or diglycerides include lauric acid, stearic acid, oleic acid, linoleic acid and linolenic acid residues. In the case of diglycerides, use may be made of a combination of two fatty acid residues selected from among the above-mentioned ones. The mono- or diglycerides may be monoglycerides, diglycerides or mixtures thereof which optionally contain triglycerides too.

Examples of the polycarboxylic acid include saturated dicarboxylic acid such as oxalic acid, malonic acid, succinic acid, glutaric acid, adipic acid, pimelic acid, suberic acid and azelaic acid; unsaturated dicarboxylic acids such as maleic acid, methylmaleic acid, fumaric acid and methylfumaric acid; hydroxypolycarboxylic acids such as malic acid, tartaric acid and citric acid; and esterified polycarboxylic acids prepared by esterifying all or some of hydroxyl groups of a hydroxycarboxylic acid with acetic acid or the above-mentioned carboxylic acids such as oxalic acid (for example, diacetyltartaric acid).

As the reactive derivatives of polycarboxylic acids, it is preferable to use acid anhydrides. Preferable examples of such acid anhydrides include diacetyltartaric anhydride, maleic anhydride, phthalic anhydride and succinic anhydride. It is particularly preferable to use diacetyltartaric anhydride or succinic anhydride therefor.

The esterification of the mono- or diglyceride with the polycarboxylic acid or a reactive derivative thereof (hereinafter referred to as "polycarboxylic acid") is not particularly restricted. It may be carried out by mixing the mono- or diglyceride with the polycarboxylic acid and treated the mixture at a temperature of 85 to 200 °C for 0.1 to 3 hours. It is preferable to use from 0.1 to 10 parts by mol of the polycarboxylic acid per part of the mono- or diglyceride.

It is also possible to carry out the esterification in the presence of an amine such as pyridine and a basic catalyst. Thus the reaction can be completed at a lower temperature within a shorter period of time.

The ester obtained by the above-mentioned reaction may contain both of the ester of the monoglyceride with the polycarboxylic acid and the ester of the diglyceride with the polycarboxylic acid. The ratio of the contents of these components is not particularly restricted.

It may further contain the unreacted polycarboxylic acid and mono- or diglyceride and polymerization products thereof. However, it is preferable to purify the reaction product so as to regulate the content of the components other than the ester of the mono- or diglyceride with the polycarboxylic acid to not more than 80 % by weight, still preferably not more than 50 % by weight and still preferably not more than 30 % by weight, and particularly preferably not more than 5 % by weight.

Furthermore, the ester of the mono- or diglyceride with the polycarboxylic acid may contain a carboxyl group having a free proton, which does not participate in the reaction, or form a salt. Examples of the counter ion for the formation of the salt include sodium, potassium, calcium, magnesium and aluminum. Sodium and potassium are particularly preferable therefor.

The content of (B) in the taste modifier preferably ranges from 0.01 to 99.0 %, still preferably from 10 to 90 %.

The weight ratio of the component (A) to the component (B) preferably ranges from 1/100 to 100/1, still preferably from 1/10 to 10/1.

The expression "taste modification" to be used herein means to relieve or regulate unpleasant tastes [for example, bitterness, pungency, puckery taste, tang, astringency (stimulating taste)] and to improve the aftertaste.

When added to medicinal compositions for oral use, foods or cosmetics having unpleasant tastes such as bitterness, the taste modifier of the present invention can relieve or improve the unpleasant tastes.

The medicinal compositions for oral use, foods and cosmetics having unpleasant tastes such as bitterness, to which the taste modifier of the present invention is to be added, may be in arbitrary forms, for example, liquids (for example, aqueous solution, suspension, emulsion), pastes or solids (for example, powder). Also, the taste modifier may be added in an arbitrary manner without restriction. When such a product is in the form of a liquid (for example, aqueous solution, suspension, emulsion) or a paste, the taste modifier of the present invention may be added thereto followed by thoroughly stirring and dispersing. The stirring and dispersing may be performed by using a homogenizer, an emulsifier, an ultrasonic treatment device, etc. The dispersion thus obtained may be dried by spray drying, freeze-drying, etc. to give a powdery or granular solid product. When the medicinal composition for oral use, food or cosmetic having unpleasant tastes is a solid matter (for example, powder), the taste modifier of the present invention may be added thereto followed by mixing. Alternatively, the above-mentioned taste modifier is dispersed in water, etc. and then mixed with the medicinal composition for oral use, food or cosmetic having bitterness, etc. in the form of a solid. Then the resulting mixture is homogenized and dehydrated. When the medicinal composition for oral use, food or cosmetic having bitterness, etc. is hardly soluble in water, it may be dissolved or dispersed with the use of an organic solvent (for example, hexane, ethyl acetate) or an alcohol (for example, ethanol) followed by the addition of the taste modifier of the present invention.

The medicinal composition for oral use having bitterness, etc., to which the taste modifier of the present invention is to be added, is not particularly restricted. Namely, any medicinal composition having bitter taste, etc. is usable therefor. Examples thereof include basic drugs such as promethazine, propranolol, berberine, chlorpromazine, chlorphenylamine, papaverine, thiamine and quinine; mineral acid salts of the basic drugs such as hydrochloride, nitrate, sulfate, acetate, citrate and carbonate; organic acid salts of the basic drugs such as maleate; Chinese orthodox drug preparations or crude drug preparations such as coptis rhizome, swertia, cinnamon bark, sophora root, *Phellodendron amurense RUPR*., safflower, rhubarb, scutellaria root, phellodendron bark, gymnema, aloe, *Ginkgo biloba L.*, chlorella and jujube.

The medicinal composition for oral use containing the taste modifier of the present invention may be in various dosage forms without restriction. Examples thereof include solid preparations such as capsules, granules, fine subtilaes, pills, dusts, tablets, freeze-dried preparations, troches, chewable tablets and dry syrups; and liquid preparations such as solutions, extracts, elixirs, spirits, syrups, aromatic waters, lemonades and fluidextracts.

These preparations may be produced by publicly known methods. For example, the taste modifier of the present invention is mixed with one or more additives (for example, fillers, binders, disintegrating agents, lubricants, fluidizing agents, coatings, corrigents, masking agents, perfumes) and treated with a granulator (for example, planetary mixer, stirring granulator, high-speed mixing granulator, extrusion granulator, fluidized bed granulator, centrifugal rolling granulator, roller compactor) and/or a spray dryer or a freeze dryer.

The content of the taste modifier of the present invention in the medicinal composition preferably ranges from 0.01 to 10 % by weight, particularly preferably from 0.01 to 5 % by weight and still preferably from 0.1 to 3 % by weight.

The food tasting bitter to which the taste modifier of the present invention is to be added is not particularly restricted. Examples thereof include citrus fruits (for example, grapefruit, orange, lemon) and fruit juices containing the same; vegetables (for example, tomato, green pepper, celery, gourd, carrot, potato, asparagus) and vegetable juices containing the same; seasonings (for example, sauce, soy sauce, *miso*, stock seasonings, red pepper); processed soybean products (for example, soybean milk); emulsified foods (for example, cream, dressing, mayonnaise, margarine); fish meat and processed fish products (for example, ground fish meat, fish roe); nuts (for example, peanut); fermented foods (for example, *natto*); meat and processed meat products; drinks (for example, beer, whisky, coffee, cocoa, tea, green tea, fermented tea, semi-fermented tea, soft drinks, functional drinks); pickles; noodles; soups including powdery soup; dairy products (for example, cheese, cow's milk); bread and cakes; confectionery (for example, snack, chewing gum, chocolate); candies; cigarettes; and health foods. It is also possible to add the taste modifier of the present invention to a flavor to be used in foods to thereby relieve the bitterness of the flavor. In some foods, bitterness serves as an important factor of the tastes. In such a case, the taste modifier of the present invention can be used to control the bitterness. It is furthermore possible to relieve the bitterness of amino acids tasting bitter (for example, leucine, isoleucine, phenylalanine), peptides and oligosaccharides by adding the taste modifier of the present invention thereto.

Moreover, the taste modifier of the present invention can be added in order to control the bitterness of a food which has a favorable bitterness. Examples of such a food include luxury drinks (for example, coffee, black tea, green tea); alcoholic drinks (for example, beer, whisky); some soft drinks (for example, vegetable juice); and spicy vegetables and root vegetables (for example, edible wild plants). The taste modifier of the present invention is also usable in the step of boiling spicy vegetables or root vegetables to remove the harsh taste.

The taste modifier of the present invention can be added to pungent foods to thereby control the pungency. These pungent foods include those which inherently taste pungent and those to which pungent components have been added.

Examples of the pungent component include substances obtained by extracting cayenne peppers (red, black and yellow ones), pepper, Japanese pepper, horseradish, onion, Japanese white radish, Welsh onion, garlic, ginger, etc. Particular examples thereof include capsaicin originating in cayenne pepper, piperine and chavicin originating in pepper, α- and β-sanshools and spilanthol originating in Japanese pepper, allyl mustard oil originating in Japanese white radish, black mustard and Japanese pepper, sinalbin mustard oil originating in white mustard, crotonyl mustard oil originating in rapeseed, phenylethyl mustard oil originating in nasturtium officinal and reseda odorata L, benzyl mustard oil originating in piper nigrum L, diallyl sulfide originating in Welsh onion and garlic, propylallyl disulfide originating in onion and garlic, diallyl sulfide originating in onion, dipropyl disulfide originating in onion, diallyl trisulfide originating in garlic, zingerone and syogaol originating in ginger, gingerol originating in ginger producted in Africa. Moreover, use can be made of pungent components comprising the above-mentioned components which have been synthesized chemically.

Examples of foods inherently containing these pungent components include solid spices, i.e., ground dry matters, (for example, mustard powder, Japanese horseradish powder, Japanese pepper powder and pepper), pasty spices (for example, mustard paste, Japanese horseradish paste, ginger paste, garlic paste), and composed spices [for example, cayenne pepper blend, curry powder, tabasco, salt/pepper, *layou* (spicy oil used to season Chinese food), mustard/*miso*, doubanjiang).

Examples of foods to which the above-mentioned pungent components have been added to impart pungency include snacks, baked cakes, noodles including instant noodles, soups including powdery soups, *misoshiru*, curry roux, sauces for grilled meat, grilled meat, *mentaiko* (salted cod roe with cayenne pepper flavor), pickles including kimchi, salted fish guts, candies, chewing gums, chocolates, vegetable juices, coffee, cocoa, black tea, green tea, fermented tea, semi-fermented tea, soft drinks, functional drinks, emulsified foods (for example, dressing, mayonnaise), processed soybean products (for example, soybean milk, *tofu*), fish meat and processed fish products (for example, ground fish meat, roasted fish), seasonings (for example, sauce, *miso*, soy sauce, ketchup), cooked rice, edible oils, bread, cakes, pasta (for example, spaghetti), nuts (for example peanut), boiled and seasoned foods, fermented foods and health foods.

It is preferable that the pungent components contained in the foods tasting pungent as described above are those selected from a group consisting of capsaicin, piperine, allyl mustard oil, α- and β-sanshools and syogaol.

The content of the taste modifier of the present invention in the food preferably ranges from 0.01 to 10 % by weight, particularly preferably from 0.01 to 5 % by weight and still preferably from 0.1 to 3 % by weight.

As described above, the taste modifier of the present invention can be added directly to foods having bitterness, pungency, puckery taste, tang, etc. to thereby regulate these unpleasant tastes. Alternatively, it may be kept in mouth optionally followed by chewing before, during or after eating a food having unpleasant tastes, thus regulating the unpleasant tastes. For example, a drink containing the taste modifier is prepared and then kept in mouth before, during or after taking a food having pungency, etc. Thus the pungency, etc. can be relieved and the irrirativeness in the oral cavity can be eliminated.

The cosmetic having bitter taste, etc. to which the taste modifier of the present invention is to be added is not particularly restricted. Examples thereof include facial cosmetics (for example, lotion, milky lotion, cream, pack, lipstick, foundation, shaving cream, after shaving lotion, cleansing foam, cleansing gel); and oral cosmetics (for example, toothpaste, mouse wash, mouse rinse).

Examples of the bitter component include surfactants (for example, sodium alkylsulfate, sodium monoalkylphosphate), flavors (for example, menthol, linalol, phenylethyl alcohol, ethyl propionate, geraniol, linalyl acetate, benzyl acetate), bactericides (for example, methylparaben, propylparaben, butylparaben), humectants (for example, lactic acid, sodium lactate), alcohol-denaturing agents (for example, 8-acetylated sucrose, brucine), and astringents (for example, aluminum lactate).

The content of the taste modifier of the present invention in the cosmetic preferably ranges from 0.01 to 10 % by weight, particularly preferably from 0.01 to 5 % by weight, still preferably from 0.1 to 3 % by weight.

Owing to its excellent taste-modifying effect, the taste modifier of the present invention can be used in foods, medicinal compositions for oral use or cosmetics having unpleasant tastes (for example, bitterness, pungency, puckery taste, tang, astringency), thus masking these unpleasant tastes and improving the aftertaste.

Further, the taste modifier of the present invention is advantageous to handling, etc., since it can be uniformly dispersed in water in a stable state.

To further illustrate the present invention in greater detail the following Examples will be given.

Unless otherwise noted, all parts given in the following Examples are by weight.

### [Preparation of lipid mixture containing phosphatidic acid or its lyso form of the component (A)]

### (Preparation of component A1)

25 g of soybeans (silky beans) made in United States were added to 150 g of a 0.1 M acetate buffer (pH 6) containing 50 mM of calcium chloride. Then the beans were ground in a moist state at ordinary temperatures and centrifuged at 3,000 rpm for 10 minutes to thereby give 120 g of a supernatant (extract). Next, 25 g of commercially available defatted lecithin (SLPW-SP™, manufactured by Tsuru Resichin Kogyo) was introduced into a four-necked flask (500 ml) provided with a stirrer and 120 g of the extract of the ground soybeans obtained above was added thereto. To the obtained mixture was added 250 ml of ethyl acetate under stirring. Further, 32.5 g of water was added thereto and the resulting mixture was allowed to react while stirring at 30 °C for 20 hours. The ethyl acetate layer was separated from the reaction product and the residual aqueous layer was extracted with chloroform/methanol (2/1, v/v) twice. The extract thus obtained was subjected to folch partition. Separately, the ethyl acetate was removed from the ethyl acetate layer to give the residue. After combining, chloroform/methanol was removed therefrom to thereby give 22 g of a lipid mixture as the product. The components of the lipid mixture thus obtained were analyzed by using a thin layer silica gel plate and detected through color-development with the use of sulfuric acid. Table 1 shows the results.

### (Preparation of component A2)

150 g of commercially available phosphatidylcholine (Epikuron, manufactured by Lucasmeyer) was introduced into a four-necked flask (5,000 ml) provided with a stirrer. After adding 1,500 ml of hexane/ethyl acetate (2/1, v/v) thereto, the resulting mixture was stirred to thereby dissolve the phosphatidylcholine. Next, 1,500 ml of an acetate buffer solution (pH 8) containing 100 U of phospholipase D with a microbial origin (originating in *Streptomyces Chromofuscus*, manufactured by Asahi Chemical Industry Co., Ltd.) was added thereto followed by the addition of 100 g of calcium chloride. The resulting mixture was allowed to react under stirring for 36 hours while maintaining the temperature at 37 °C. After the completion of the reaction, the solvent layer was separated and the solvent was eliminated under reduced pressure. Thus about 100 g of a lipid mixture was obtained as a residue. Similar to the above-mentioned case, the components of the lipid mixture was analyzed by using a thin layer silica gel plate and detected by color-development with sulfuric acid. Table 1 shows the results.

### (Preparation of component A3)

To 100 g of soybean phospholipid (SLP Paste, manufactured by Tsuru Resichin Kogyo) was added 500 ml of 99.5 % ethanol maintained at 45 °C. The obtained mixture was extracted by stirring with a disperser (manufactured by Janke & Kunkel) at 9,500 rpm for 10 minutes followed by filtration through a glass filter. Next, the precipitate was subjected to extraction thrice in total and the solvent was removed therefrom with the use of a vacuum dryer to thereby give the component A3. A thin layer silica gel plate (Kieselgel, manufactured by Merck, Inc.) was used in the analysis while color development with sulfuric acid was carried out for the detection. Table 1 shows the results.

**Table 1**

| Component of lipid mixture | Component A1 (%) | Component A2 (%) | Component A3 (%) |
|---|---|---|---|
| (neutral phospholipid) | | | |
| PC | 0 | 0 | 0.6 |
| PE | 0 | 0 | 10.9 |

| (acidic phospholipid) | | | |
|---|---|---|---|
| PI | 0 | 0 | 25.8 |
| PA | 62.0 | 94.0 | 23.1 |
| PS | 0.1 | 0 | 0 |
| L-PA | 0.9 | 1.0 | 0.5 |
| others | 37.0 | 5.0 | 39.1 |
| PC: phosphatidylcholine | | | |
| PE: phosphatidylethanolamine | | | |
| PI: phosphatidylinositol | | | |
| PA: phosphatidic acid | | | |
| PS: phosphatidylserine | | | |
| L-PA: lysophosphatidic acid. | | | |

### EXAMPLE 1

The component A1 was added at a ratio of 1 % w/v to a liquid preparation containing 5 % w/v of a protein decomposition product (decomposed casein) tasting bitter (control).

To the above-mentioned liquid preparation was further added 0.1 % w/v of diglyceride succinate having a fatty acid residue carrying 12 carbon atoms. The resulting mixture was vigorously stirred in a disperser for 5 minutes to thereby give a taste modifier (Example 1).

### EXAMPLE 2

The procedure of the above Example 1 was repeated but substituting the diglyceride succinate by monoglyceride succinate (Step SS, manufactured by Kao Corporation). Table 2 shows the data on dispersibility and bitterness intensity.

Regarding the dispersibility, a solution regarded as transparent with the naked eye was referred to as "good".

### (Bitterness intensity)

To evaluate the bitterness intensity, a sensory test was effected with the use of 10 male and female panelists of twenties to forties in age. The bitterness was evaluated in 5 grades in accordance with the following criteria and expressed in the average.
5: very bitter.
4: bitter, but not so strong.
3: slightly bitter.
2: noticeably bitter.
1: not bitter.

**Table 2**

| | Bitterness intensity | Dispersibility |
|---|---|---|
| component A1 | 3.3 | opaque |
| component A1 + diglyceride succinate | 2.4 | good |
| component A1 + Step SS | 3.0 | good |

Each of the liquid preparations obtained in Examples 1 and 2 showed a controlled bitterness and a high dispersibility.

### EXAMPLE 3

To 3 coffee drinks containing milk components was added 0.05 % w/v of the component A2 or the component A3 (control). At the same time, 0.01 % w/v of sodium salt of diglyceride succinate or sodium salt of monoglyceride succinate, each having a fatty acid residue carrying 12 carbon atoms, was added thereto to thereby give a coffee drink containing a taste modifier. Table 3 shows the results of the flavor evaluation of these products.

**Table 3**

| | Instant coffee with creaming powder | Regular coffee with creaming powder | Milk with coffee flavor |
|---|---|---|---|
| component A2 or A3 | relieved miscellaneous taste | relieved bitterness | relieved miscellaneous taste |
| component A2 or A3 + Na salt of diglyceride succinate | relieved miscellaneous taste, body and mild flavor | relieved bitterness, mild flavor | relieved miscellaneous taste, body flavor |
| component A2 or A3 + Na salt of monoglyceride succinate | relieved miscellaneous taste, body flavor | relieved bitterness, mild flavor | relieved miscellaneous taste, body flavor |

Thus coffee drinks with improved flavor could be obtained.

### EXAMPLE 4

By using the composition 1 as specified below, nucleated granules were produced by using a high speed mixer. After drying and classifying, these granules were coated with an aqueous solution of the composition 2 as specified below by fluidized bed spraying to thereby give a coated granule preparation B.

| Composition | Part by weight |
|---|---|
| (composition 1) | |
| propranolol (active component) | 1 |
| corn starch | 35 |
| lactose | 54.5 |
| hydroxypropylcellulose (HPC) | 3.5 |

| (composition 2) | |
|---|---|
| component A3 | 3 |
| hydroxypropylcellulose (HPC) | 1.5 |
| diglyceride succinate having a fatty acid residue carrying 12 carbon atoms | 1.5 |
| | 100 |

Further, a control product was produced by the same method except using no diglyceride succinate but 1 part by weight of the component A3 and 5 parts by weight of HPC. Table 4 shows the data on bitterness intensity and dispersibility of the component A3.

**Table 4**

| | Bitterness intensity | Dispersibility |
|---|---|---|
| control | 3.3 | partly heterogenous |
| coated granule preparation B | 2.6 | homogeneous |

### EXAMPLE 5

By adding 1.0 % of the component A3 and 0.1 % of diglyceride succinate to a commercially available curry product (hot type), a product having a relieved pungency and a mild flavor could be obtained. Although the obtained product was less pungent in mouth, it suffered from no change in the body-warming effect after taking characteristic to hot curry.

## Claims

1. A taste modifier which comprises the following components (A) and (B):
(A) an acidic phospholipid or a lyso form of the same or a lipid mixture containing said acidic phospholipid or a lyso form of the same, wherein the content of the acidic phospholipid or its lyso form in the taste modifier is in the range from 1 to 99% by weight; and
(B) an ester of mono- or diglyceride with a polycarboxylic acid.

2. A taste modifier as claimed in claim 1, wherein said component (A) is a phosphatidic acid or a lysophosphatidic acid.

3. A taste modifier as claimed in claim 1, wherein the weight ratio of said component (A) to said component (B) ranges from 100/1 to 1/100.

4. A medicinal composition for oral use which contains a taste modifier as claimed in any of claims 1 to 3.

5. A food which contains a taste modifier as claimed in any of claims 1 to 3.

6. A cosmetic which contains a taste modifier as claimed in any of claims 1 to 3.

7. Use of a combination of
(A) an acidic phospholipid or a lyso form of the same or a lipid mixture containing said acidic phospholipid or a lyso form of the same, wherein the content of the acidic phospholipid or its lyso form in the taste modifier is in the range from 1 to 99% by weight; and
(B) an ester of mono- or diglyceride with a polycarboxylic acid
as a taste modifier.

## Patentansprüche

1. Geschmacksmodifizierungsmittel, welches die folgenden Komponenten (A) und (B) umfasst:
(A) ein saures Phospholipid oder die Lysoform davon oder ein Lipidgemisch, das das saure Phospholipid oder eine Lysoform davon enthält, wobei der Gehalt an saurem Phospholi pid oder seiner Lysoform in dem Geschmacksmodifizierungsmittel im Bereich von 1 bis 99 Gew.-% liegt; und
(B) einen Ester eines Mono- oder Diglycerids mit einer Polycarbonsäure.

2. Geschmacksmodifizierungsmittel nach Anspruch 1, wobei die Komponente (A) eine Phosphatidsäure oder eine Lysophosphatidsäure ist.

3. Geschmacksmodifizierungsmittel nach Anspruch 1, wobei das Gewichtsverhältnis der Komponente (A) zu der Komponente (B) im Bereich von 100/1 bis 1/100 liegt.

4. Medizinisches Präparat für die orale Verwendung, welches ein Geschmacksmodifizierungsmittel nach einem der Ansprüche 1 bis 3 enthält.

5. Nahrungsmittel, welches ein Geschmacksmodifizierungsmittel nach einem der Ansprüche 1 bis 3 enthält.

6. Kosmetikum, welches ein Geschmacksmodifizierungsmittel nach einem der Ansprüche 1 bis 3 enthält.

7. Verwendung einer Kombination aus
(A) einem sauren Phospholipid oder der Lysoform davon oder eines Lipidgemisches, das das saure Phospholipid oder eine Lysoform davon enthält, wobei der Gehalt des sauren Phospholipids oder seiner Lysoform in dem Geschmacksmodifizierungsmittel im Bereich von 1 bis 99 Gew.-% liegt; und
(B) einem Ester aus Mono- oder Diglycerid mit einer Polycarbonsäure
als Geschmacksmodifizierungsmittel.

## Revendications

1. Modificateur de goût comprenant les composants (A) et (B) suivants:
(A) un phospholipide acide ou une forme lyso de celui-ci ou un mélange de lipides contenant ledit phospholipide acide ou une forme lyso de celui-ci, le taux du phospholipide acide ou de la forme lyso de celui-ci dans le modificateur de goût étant dans la plage de 1 à 99 % en masse et
(B) un ester de mono- ou diglycéride avec un acide polycarboxylique.

2. Modificateur de goût selon la revendication 1, dans lequel ledit composant (A) est un acide phosphatidique ou un acide lysophosphatidique.

3. Modificateur de goût selon la revendication 1, dans lequel le rapport pondéral dudit composant (A) audit composant (B) se situe dans la plage de 100/1 à 1/100.

4. Composition médicinale pour une utilisation orale qui contient un modificateur de goût selon l'une quelconque des revendications 1 à 3.

5. Aliment qui contient un modificateur de goût selon l'une quelconque des revendications 1 à 3.

6. Cosmétique qui contient un modificateur de goût selon l'une quelconque des revendications 1 à 3.

7. Utilisation d'une combinaison
(A) d'un phospholipide acide ou d'une forme lyso de celui-ci ou d'un mélange de lipides contenant ledit phospholipide acide ou une forme lyso de celui-ci, le taux du phospholipide acide ou de la forme lyso de celui-ci dans le modificateur de goût étant dans la plage de 1 à 99 % en masse et
(B) d'un ester de mono- ou diglycéride avec un acide polycarboxylique.
en tant que modificateur de goût.
